Europäisches Patentamt

European Patent Office

Office européen des brevets

(11) Veröffentlichungsnummer: **0 287 051**
**A2**

## (12) EUROPÄISCHE PATENTANMELDUNG

(21) Anmeldenummer: 88105858.0

(22) Anmeldetag: **13.04.88**

(51) Int. Cl.⁴: **G01N 33/00 , G01N 30/00**

(30) Priorität: 16.04.87 DE 3712924

(43) Veröffentlichungstag der Anmeldung:
**19.10.88 Patentblatt 88/42**

(84) Benannte Vertragsstaaten:
**DE ES FR GB IT NL**

(71) Anmelder: **Drägerwerk Aktiengesellschaft**
**Moislinger Allee 53-55**
**D-2400 Lübeck 1(DE)**

(72) Erfinder: **Evers, Wolfgang**
**Koggenweg 3**
**D-2400 Lübeck(DE)**

(54) **Plakettenförmiges Dosimeter mit gewölbtem Substratträger.**

(57) Ein Dosimeter zum Nachweis von Schadstoffen, die auf einem plakettenförmigen Substratträger gesammelt sind, der mit einer ihm anliegenden Diffusionsmembran gegenüber der Umgebung abgedeckt ist, soll so verbessert werden, daß eine über die ganze Auflagefläche der Membran auf dem Substratträger gleichmäßige Anlage erzielt werden kann. Dazu ist vorgesehen, daß der Substratträger (4) zur Umgebung hin gewölbt ausgebildet ist.

EP 0 287 051 A2

## Plakettenförmiges Dosimeter mit gewölbtem Substratträger

Die Erfindung betrifft ein Dosimeter zum Nachweis von Schadstoffen, die auf einem plakettenförmigen Substratträger gesammelt sind, der mit einer ihm anliegenden Diffusionsmembran gegenüber der Umgebung abgedeckt ist.

Aus der DE-OS 27 36 975 ist ein derartiges Dosimeter bekannt geworden, bei welchem in einem Gehäuse in einer Vertiefung ein teilchenförmiges Adsorptionsmaterial zur Sammlung des nachzuweisenden Schadstoffes eingefüllt ist. Dieser plakettenförmige Substratträger ist mit einer Membran abgedeckt, die für die nachzuweisenden Dämpfe durchlässig ist, die dann in das Adsorptionsmaterial eindringen und deren Vorhandensein durch eine nachfolgende beispielsweise naßchemische Nachweismethode festgestellt werden kann.

Benutzt man derartige Plaketten statt mit dem bekannten Adsorptionsmaterial mit einem kolorimetrischen Indikator, kommt es wesentlich darauf an, daß die über den Substratträger aufgelegte permeable Membran über ihren gesamten Abdeckungsbereich einen gleichmäßigen Abstand bzw. ständig berührend dicht an den Substratträger anliegt. Unterschiedliche Abstände haben auch unterschiedliche Diffusionsweglängen zur Folge, so daß unterschiedliche Farbtönungen entstehen, die in Wirklichkeit nicht auf unterschiedliche Konzentrationen der nachzuweisenden Schadstoffe zurückzuführen sind. Es ist daher beim Bespannen der Dosimeter höchste Sorgfalt darauf zu legen, daß die Membranen gleichmäßig eng und in gleichbleibendem Abstand zum Substratträger anliegen, was bei den kleinen Dimensionen und der Empfindlichkeit der Membranen nicht immer gewährleistet ist. Ein sorgfältiges Bespannen kann trotzdem zu Faltenbildung in der Membran führen. Das Festspannen der Membran an ihrem Randbereich führt dazu, daß dort zunächst der höchste Anpreßdruck auf den Substratträger entsteht, welcher zur Mitte der Membran hin stetig abnimmt und somit zu einem Druckgradienten führt, der über den gesamten Verlauf der Membran zu einer unterschiedlichen Anpreßkraft auf den Substratträger führt. Auch dies führt zu einem unterschiedlichen Diffusionsverhalten des nachzuweisenden Gases aus der Umgebung in den Substratträger, abgesehen davon, daß das übermäßige Anspannen und Dehnen der Membran örtlich unterschiedliches Diffusionsverhalten des Gases innerhalb der Membran zur Folge hat.

Der vorliegenden Erfindung liegt somit die Aufgabe zugrunde, ein Dosimeter der genannten Art so zu verbessern, daß eine über die ganze Auflagefläche der Membran auf dem Substratträger gleichmäßige Anlage erzielt werden kann.

Die Lösung der Aufgabe erfolgt dadurch, daß der Substratträger zur Umgebung hin gewölbt ausgebildet ist.

Die mit der Erfindung erzielten Vorteile liegen im wesentlichen darin, daß nunmehr die Membran über die Wölbung gezogen werden kann, und daß keine Hohlräume bzw. unterschiedliche Abstände zum Substratträger entstehen können. Der Anlagedruck der Membran braucht jetzt nicht mehr, wie bei der ebenen Ausführungsform des Substratträgers erforderlich, durch eine übermäßige Zugspannung am Randbereich der Membran aufgebracht zu werden, sondern sie bildet sich in gemäßigter Form von selbst durch das Auflegen der Membran auf die Wölbung des Substratträgers aus.

Zweckmäßigerweise wird die Membran lediglich an ihrem Randbereich im Gehäuse des Dosimeters eingespannt, so daß sie sich an die Form der Wölbung anschmiegt.

Eine besonders vorteilhafte Ausbildungsform ist darin zu sehen, daß der Substratträger zunächst eine Unterlage darstellt, die unter einer inneren Spannung steht, wobei die Membran über den Substratträger gelegt und an ihrem Randbereich festgehalten wird. Sodann ist durch einen Druck auf der Unterseite des Substratträgers die Wölbungsform herzustellen, so daß die vorher lose aufliegende Membran unter eine gleichmäßige Zugspannung versetzt wird, wodurch die Membran ohne Faltenbildung und über ihren gesamten Flächenbereich gleichmäßig dem Substratträger anliegt.

Ein Ausführungsbeispiel der Erfindung wird anhand der schematischen Zeichnung dargestellt und im folgenden näher erläutert.

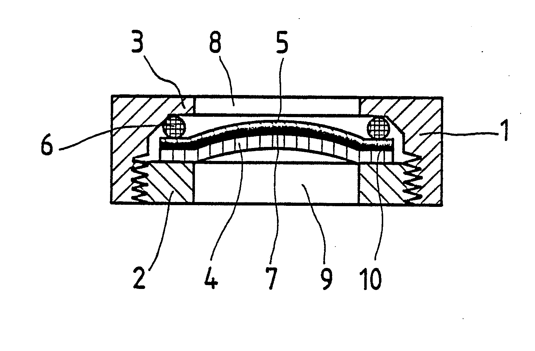

Die einzige Figur zeigt im Schnitt ein Dosimeter, welches aus einem Gehäuse (1) besteht, in welches ein Trägerring (2) eingeschraubt ist. Zwischen dem Haltekragen (3) des Gehäuses (1) sind ein Substratträger (4) und eine Diffusionsmembran (5) an ihrer Umrandung (10) mit Hilfe eines O-Ringes (6) eingespannt. Auf dem Substratträger (4) befindet sich eine für den nachzuweisenden Schadstoff empfindliche kolorimetrische Indikatorschicht (7). Zur Umgebung hin besitzt das Gehäuse (1) eine Durchtrittsöffnung (8), von der aus der nachzuweisende Schadstoff in die Diffusionsmembran (5) auf die Indikatorschicht (7) auftrifft. Die Unterseite des Substratträgers (4) ist über eine Trägerringöffnung (9) zugänglich. Von dieser Seite aus kann der ursprünglich unter einer inneren Spannung stehende Substratträger (4) in die dargestellte Wölbungsform gebracht werden, indem auf seine Unterseite ein Druck ausgeübt wird.

Der Substratträger (4) besteht aus einer Unterlage, welche über den Trägerring (2) gehalten ist.

Der Substratträger (4) kann aber auch mit dem Trägerring (2) einstückig ausgebildet sein. Des weiteren kann auch die Trägerringöffnung (9) entfallen, sofern die Wölbungsform des Substratträgers (4) von vornherein in ihn eingearbeitet ist.

## Ansprüche

1. Dosimeter zum Nachweis von Schadstoffen, die auf einem plakettenförmigen Substratträger gesammelt sind, der mit einer ihm anliegenden Diffusionsmembran gegenüber der Umgebung abgedeckt ist, dadurch gekennzeichnet, daß der Substratträger (4) zur Umgebung hin gewölbt ausgebildet ist.

2. Dosimeter nach Anspruch 1, dadurch gekennzeichnet, daß die Diffusionsmembran (5) an der planen Umrandung (10) des Substratträgers (4) im Dosimetergehäuse (1) eingespannt ist.

3. Dosimeter nach einem der Ansprüche 1 und 2, dadurch gekennzeichnet, daß der Substratträger aus einer unter einer inneren Spannung stehenden Unterlage (4) besteht, die nach ihrer Abdeckung mit der Diffusionsmembran (5) in die konvexe Wölbungsform durch Druckausübung auf ihre der Membran (5) abgewandten Unterseite überführbar ist.